(19) **Europäisches Patentamt**

**European Patent Office**

**Office européen des brevets**

(11) **EP 1 172 087 A2**

(12) **EUROPEAN PATENT APPLICATION**

(43) Date of publication:
**16.01.2002 Bulletin 2002/03**

(51) Int Cl.⁷: **A61K 7/48**, A61P 17/10

(21) Application number: **01306021.5**

(22) Date of filing: **12.07.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.07.2000 AU PQ877300**

(71) Applicant: **Johnson & Johnson Pacific Pty Limited**
**Botany, New South Wales 2019 (AU)**

(72) Inventors:
• **Gomes, Anna**
  **New South Wales 2203 (AU)**
• **Khaiat, Alain V.**
  **Sinapore, 437991 (SG)**
• **Bhide, Vaishali**
  **Kalyan 421304, Dist Thane, Maharashtra (IN)**

(74) Representative: **Fisher, Adrian John et al**
**CARPMAELS & RANSFORD 43 Bloomsbury Square**
**London WC1A 2RA (GB)**

(54) **Topical treatment of skin**

(57)    The invention relates to the use as a sebum regulating agent of a hydrolysed vegetable protein produced by enzymatic hydrolysis, and to compositions comprising a hydrolysed vegetable protein produced by enzymatic hydrolysis for application to the skin to inhibit or regulate sebum production, to inhibit or treat oily skin, or to prevent, inhibit or treat acne. The vegetable protein may be for example a cereal protein such as soy protein, wheat protein or an extract from a tree such as cedar, poplar or mimosa, or from the foliage or from the various stages of the flower, such as the bud. Preferably the vegetable protein has a molecular weight in the region of 100,000 to 500,000 Daltons.

The invention also relates to methods of preventing, controlling or inhibiting the oily/shiny appearance and nature of skin and consequential disorders such as acne, comprising the topical application of a hydrolysed vegetable protein, produced by enzymatic hydrolysis, to the skin.

**EP 1 172 087 A2**

**Description**

This invention relates to the treatment of skin and more particularly to the treatment of conditions of skin caused through excess sebum production and the consequences thereof such as acne.

Excess sebum production is a common problem particularly with teenagers leading to an oily/shiny appearance of the skin which in itself causes embarrassment and is also one of the principal factors causing acne. The oily/shiny appearance of skin results from excess sebum excretion in the sebaceous glands. The classic approach to addressing oily/shiny skin is the use of powders that provide an immediate masking effect by absorbing the excess sebum on the skins surface. Such an approach has short term benefit only and has a minimal effect on conditions such as acne caused through excess sebum production.

More recently, topical agents have been studied and found to have activity as oil controlling agents. One of these is elubiol (dichlorophenyl-imidazoltioxolan). Elubiol is an effective oil control agent. Regulatory approval is being sought for its use for this purpose.

There is a need for alternative sebum regulating agents. This invention provides a number of different products which have sebum regulating effects.

In a first aspect, the invention provides the use as a sebum regulating agent of a hydrolysed vegetable protein produced by enzymatic hydrolysis.

This aspect of the invention also provides compositions for application to the skin to inhibit or regulate sebum production, to inhibit or treat oily skin, to prevent or inhibit the development of acne and to treat acne when present comprising a hydrolysed vegetable protein produced by enzymatic hydrolysis.

This aspect also includes a method of preventing, controlling or inhibiting the oily/shiny appearance of skin and consequential disorders resulting therefrom such as acne comprising the topical application of a hydrolysed vegetable protein, such as soy protein, produced by enzymatic hydrolysis to the skin.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to".

Preferably the vegetable protein is a cereal protein such as soy protein. Hydrolysed soy protein produced by enzymatic hydrolysis can be obtained from Maybrook Inc under the trade mark SOY-TEIN NL.

Hydrolysed soy protein can be produced by other methods such as by bacterial fermentation. Hydrolysed proteins produced by bacterial fermentation are clearly distinguishable from those produced by enzymatic hydrolysis and are not included within this aspect of the invention.

Such compositions can include other active agents designed to assist in improving skin appearance and assist in inhibiting the development of other conditions, such as acne, such as keratolytic agents for example salicylic acid, benzoyl peroxide, resorcinol, colloidal sulphur, selenium disulphide, sulfur and anti-inflammatory agents such as alpha-bisabolol, dipotassium glycyrrhizinate, allantoin, matricaria (chamomilla recutita) extract, tocopheryl acetate, green tea (camellia sinesis) extract, turmeric (curcuma longa) extract.

The compositions can contain other ingredients normally present in formulations for skin application as will be elaborated below in discussing compositions for use in all aspects of the invention.

In a second aspect of the invention there is provided the use of hydrolysed cereal proteins, such as wheat proteins, produced by any suitable method, for controlling, or at least inhibiting the oily/shiny appearance of skin and any consequential effects thereof such as acne.

This aspect of the invention also includes compositions for use in controlling or at least inhibiting the oily nature of skin, and inhibiting, or controlling, consequences thereof such as acne, containing the hydrolysed cereal protein such as wheat protein, and suitable carriers.

This aspect also includes a method of controlling or at least inhibiting, the oily nature of skin and consequences thereof including acne, comprising the topical application of a composition containing hydrolysed cereal protein, such as wheat protein, to the affected part of the skin. The invention also includes a method of treating acne or at least inhibiting it, and a method of preventing the development of oily skin, by applying hydrolysed cereal protein, such as wheat protein, to skin susceptible to developing excess oiliness.

The hydrolysed cereal protein can be a hydrolysed wheat protein, produced by any hydrolysis method such as soluble wheat proteins, preferably of a high molecular weight type having a molecular weight in the region of 100,000 to 500,000 Daltons, but lower molecular weight hydrolysates are also believed to be effective. High molecular weight products sold by Croda Inc such as Tritisol having a molecular weight of 100,000 Daltons and Tritisol XM having a molecular weight of 500,000 Daltons are particularly suitable.

In addition the compositions can include keratolytic agents such as salicylic acid, benzoyl peroxide, resorcinol, colloidal sulphur, selenium disulphide, sulfur and anti-inflammatory agents such as alpha-bisabolol, dipotassium glycyrrhizinate, allantoin, matricaria (chamomilla recutita) extract, tocopheryl acetate, green tea (camellia sinesis) extract, turmeric (curcuma longa) extract.

The compositions can include other ingredients normally present in formulations for topical application to the skin as discussed below in relation to all aspects of the invention.

In a third aspect of the invention there is provided the use for preventing, inhibiting or treating oily skin and the consequences thereof, including acne, and for inhibiting, preventing or treating acne itself, of a combination of a sebum controlling agent and a keratolytic agent, applied sequentially or simultaneously.

This aspect of the invention also includes compositions for such use including a sebum regulating agent and a keratolytic agent.

This aspect of the invention also includes a method for inhibiting, treating or preventing oily skin and the consequences thereof, such as acne, and for inhibiting, treating or preventing acne comprising the application to the affected area of the skin, or the area susceptible to be affected, sequentially or simultaneously of a sebum regulating agent and a keratolytic agent.

In this third aspect of the invention the sebum regulating agent can be that derived from a natural source such as from a plant in particular hydrolysed vegetable protein such as hydrolysed cereal protein, in particular, hydrolysed wheat protein, or from other plants such as hydrolysed soy protein produced by any means such as acid, bacterial or enzymatic hydrolysis. Certain plant extracts are also included within the scope of the invention, such as extracts from suitable trees for example cedar, poplar and mimosa. Such extracts can be from the foliage or from the various stages of the flower of the particular tree, in particular from the bud.

The keratolytic agent can be any suitable agent, benzoyl peroxide, resorcinol, colloidal sulphur, selenium disulphide, sulfur, more preferably because of its effectiveness and mildness, salicylic acid.

In a fourth aspect there is provide the use of a sebum regulating agent and a deposition enhancer for preventing, inhibiting or controlling the oily/shiny appearance of skin, and/or the consequences thereof such as acne.

In this fourth aspect there is also provided a topical composition for such use comprising a sebum regulating agent and a deposition enhancer together with a suitable carrier. The fourth aspect also provides a method for preventing or at least inhibiting oily skin and/or the consequences thereof such as acne, comprising the topical application of a sebum regulating agent and a deposition enhancer such as phytantriol, polyquaternium-6, -7, -22 and -39. Preferably the deposition enhancer is phytantriol.

In accordance with a fifth aspect of the invention there is provided a method of controlling the oily/shiny appearance of skin comprising applying to the skin having such appearance or susceptible to such disorder, a lipase inhibiting substance. This fifth aspect also provides a topical composition for use in such a method comprising a lipase inhibitor and a suitable carrier.

Lipase inhibition is believed to be a mechanism by which the hydrolysed vegetable proteins such as hydrolysed soy protein and hydrolysed wheat protein and the plant extracts such as those from cedar and poplar achieve the oil control at least in part. Application of an agent to inhibit lipase activity is believed to be a novel approach to controlling oily skin.

Without wishing to be bound by any theory, it is believed that the activity of the oil control agents of the current invention in all its aspects, modulate the rate of sebum production through the follicular reservoir and through inhibiting lipase activity, or possibly also at the sebum synthesis step.

It is believed that acne is a result of a number of factors. We now understand that sebum production occurs in the sebaceous glands through the presence of 5-alpha-reductase enzyme. This enzyme is sensitive to the level of testosterone penetrating the sebaceous cell. The testosterone is transformed to dihydrotestosterone under the influence of the 5-alpha-reductase enzyme thereby leading to an abundance of sebum. Sebum consists of a mixture of squalane wax esters, cholesterol esters, and triglycerides. An abnormally high rate of sebum supports the growth and proliferation of Propionibacterium acne which degrades sebum triglycerides to diglycerides, monoglycerides and free fatty acids. The free fatty acids peroxidise in the presence of free radicals and this leads to the oily appearance, inflammation, commadones and other acne manifestations. Hence by inhibiting the lipase activity, the oiliness and consequences thereof of the skin may in turn be inhibited even where sebum production is not simultaneously controlled.

In accordance with the invention, the active ingredients for controlling the oiliness of the skin, are applied in an amount of between 2 and 4 $\mu$l/cm$^2$, preferably about 3$\mu$l/cm$^2$. The active ingredients can be applied at intervals to achieve effective results. Desirably application will be at least once a day, more preferably twice a day. Treatment periods will depend on the severity of the condition and also whether the active ingredient is being applied as a preventative measure for the development of oily skin or after oily skin has emerged or the more serious acne manifestation exists. Because the active ingredients of the invention are found to be mild and non-aggressive agents for treating these disorders of the skin, application will need to be for a significant period of time. This time may vary from person to person. Trials have shown that significant reduction in oily appearance of the skin can occur after twelve weeks.

The active ingredients of the invention in all its aspects will be applied in topically applicable compositions. The compositions can be applied on skin directly without any other preparation. It is believed that the active ingredients will work more quickly if the skin is thoroughly cleaned for application of the active ingredients, for a period of from one day up to two weeks prior to commencement of application of the active agent. A suitable wash out conditioning material is that supplied by Johnson & Johnson under the trade mark Clean & Clear Facial Wash. During application of the active ingredient, the face is washed and then thoroughly dried before application of the active agent in the topical formulation. The topical formulation, dependent on its nature, can be simply applied with a finger or through incorporation in a suitable substrate such as a suitable fabric.

The topical formulations of the invention can be in any desired form such as a gel, cream, lotion, liquid or atomiser spray. These compositions can contain other agents which have an oil control or other useful effect in the complex system of excess oiliness and the consequences thereof such as acne. These agents should not interfere with the effectiveness of the active agents of the current invention.

Compositions will include, at least in the third aspect, a keratolytic agent. The keratolytic agent is present in an effective amount usually, at least 0.1%, preferably at least 0.2%, more preferably at least 0.3% and most preferably at least 0.5%. The maximum amount will be limited generally by cost factors as excess will be unnecessary to achieve the required result and may lead to unwanted side-effects. Generally, the maximum amount will be about 2%.

The active agents of the invention are present in the topical compositions in an amount to achieve the desired result. The higher the concentration, the more rapid the desired effect will be achieved but above certain levels dependent on the particular product, increased activity becomes marginal and additional active agent is therefore wasteful. Generally observable effects can be achieved at 0.1% but more preferably the active ingredient is present at least 0.5%. The preferred maximum amount is 5% with the most preferred being 3%.

Anti-inflammatory agents are a preferred additives to the compositions of all aspects of the invention. Any suitable topical anti-inflammatory agent can be used in accordance with this invention. Preferred for their effectiveness, availability and regulatory approval status are allantoin or alpha-bisabolol. These agents will be present in effective amounts and again the amount will depend on the effectiveness of the particular substance. Effective results will generally be achievable between 0.2 and 2%, more preferably between 0.2 and 1%.

Another desirable ingredient is a deposition enhancer such as phytantriol and polyquaternium-6, -7, -22 and -39. This will be present in an effective amount generally between 0.1 and 0.5%, more preferably between 0.1 and 0.3%.

Another desirable component of the compositions is a skin penetrant substance such as propylene glycol or transcutol. The penetrant assists in ensuring the compositions of the invention penetrate to the pores of the skin to achieve the desired result.

The compositions will contain other components, normally present in skin treatment compositions such as thickeners, emulsion stabilisers, emulsifiers, emollients, occlusive agents, skin conditioners, moisturisers, humectants, preservatives, antioxidants, pH adjusting agents, surfactants, chelating agents, tackifying agents and fragrances etc. Desirably the compositions are aqueous based. Since some of the ingredients are not water miscible, the compositions will need to be formed into an emulsion using suitable emulsifying apparatus as is well known in the art, or as water miscible organic solvent added to dissolve the water immiscible ingredients.

Thickeners include suitable polymers such as Carbomer, hydroxypropyl methylcellulose, PVM/MA decadiene cross-polymer and Acrylates/$C_{10-30}$ Alkyl Acrylate cross-polymer in an amount generally between 0.15 to 1.5%, more preferably 0.45 to 1.3%, most preferably 0.15 to 1%. Two or more of such thickeners can be added. In some cases the thickeners have other effects such as being emulsion stabilisers. Other specific emulsion stabilisers may also be added. A preferred combination is the PVM/MA decadiene cross-polymer and the Acrylates/$C_{10-30}$ Alkyl Acrylate cross-polymer. PVM/MA decadiene is usually present in an amount between 0.15 to 0.5%, more preferably between 0.15 to 0.3%. Acrylates/$C_{10-30}$ Alkyl Acrylate cross-polymer is usually present between 0.3 to 0.8%, more preferably between 0.5 to 0.7%.

Another desirable ingredient is an emollient, such diisopropyl adipate/isohexadecane dimethicone and $C_{12-15}$ alkyl benzoates, generally between 2 to 5%, more preferably from 3 to 5%.

Skin conditioners such as occlusive agents for example cyclomethicone, trimethylsiloxysilicate, glycereth-26 or polyquaternium-7 (which also functions as a film former) can be included generally in an amount of between 1 to 4%, more preferably between 1 to 3%.

Emulsifiers can be added such as cetyl alcohol, stearyl, stearic acid, glyceryl stearate, propylene glycol isostearoyl-sodium isostearoyl, a lactylate, polyoxyethylene (100) stearate.

Moisturisers such as panthenol can be included generally in amount between 0.25 to 1%.

Antioxidants can also be included such as tocopheryl acetate or BHT, generally in an amount between 0.1 to 1%, more preferably between 0.2 and 1%. Tocopheryl acetate if used also has anti-inflammatory properties and hence can be present for that purpose, but desirably other anti-inflammatory agents will also be present.

Humectants can also be present such as propylene glycol or glycerin generally in an amount between 1 to 5%, more preferably between 3 and 5%.

Preservatives are desirably present such as phenoxyethanol and parabens generally in an amount between 0.5 to 1%, more preferably between 0.8 and 1%.

A pH adjusting agent which will normally be a base such as triethanolamine or sodium hydroxide in an amount sufficient to provide the desired pH which will normally be between 5.5 and 6.5. This would normally be within the range of 0.3 to 2% depending on the acidity of the remaining ingredients.

A suitable fragrance will normally be added in an amount sufficient to give the desired pleasant aroma.

The compositions can also contain a chelating agent such as disodium EDTA or sodium citrate in an amount generally between 0.01 and 0.1%, more preferably about 0.05%.

The compositions can also include detackifiers such as aluminium starch octenyl succinate in an amount generally between 1 and 2% preferably about 1.5%.

The compositions can be in the form of a liquid with an aqueous base and a suitable organic solvent miscible with water to solubilise the lipophilic ingredients. A suitable solvent for that purpose is butylene glycol. Desirably a solubiliser such as polysorbate-20 is also included.

The compositions of the invention can also have an additional cleansing effect. Such cleansing compositions in addition to the other ingredients can include surfactants such as lauryl phosphate in an amount generally between 2 to 6%, more preferably between 3 to 5%, and a foam booster in an amount between 2 to 4%, more preferably between 2.5 and 3.5% such as cocamido propyl betaine; antibacterial agents can also be included such as triclosan in an amount generally between 0.1 and 0.5% preferably about 0.25%; and

cleansing agents such as lauric acid and myristic acid are also desirably present generally in an amount between 5 and 15%, more preferably between 8 to 12%, most preferably between 9 to 10%; and

The hydrolysed soy protein of this invention produced by bacterial fermentation is supplied by Sederma under the trade mark Biodermine. It is a clear pale yellow liquid with a characteristic odour. The commercial product contains the hydrolysed soy protein and propylene glycol.

Hydrolysed wheat protein can be obtained from Croda as referred to above. It is a viscous amber solution with a characteristic odour. This is obtained by enzymatic hydrolysis. The product is a mixture of the hydrolysed wheat protein in water.

The cedar wood extract and the poplar bud extract are both obtainable from Alban Muller International. The cedar wood extract is a brownish very dark greenish liquid extract from <u>Cedrus atlantica</u>. It is understood these extracts are water soluble and obtained using propylene glycol and water as the extracting solvents. It is believed that other solvents can be used to obtain extracts which will contain agents effective to control sebum in accordance with this invention.

The poplar bud extract is a brown coloured liquid extracted from populus nigra with a balsamic odour. Again it is believed the extract so obtained uses propylene glycol and water as the extracting solvents but other extracting solvents are considered to be useful to obtain effective agents for use in this invention.

The following examples illustrate the invention.

## EXAMPLE 1

### (Gel Compositions used in trials)

| CTFA Name | Composition #1 Hydrolysed Soy Protein % w/w | Composition #2 Hydrolysed Wheat Protein % w/w | Composition #3 Poplar Bud % w/w | Composition #4 Cedarwood % w/w | Composition #5 Elubiol (Dichlorophenyl Imidazoldioxolan) %w/w | Function |
|---|---|---|---|---|---|---|
| Purified Water | Qs | Qs | Qs | Qs | Qs | vehicle |
| PVM/MA Decadiene Crosspolymer | 0.60 | 0.60 | 0.60 | 0.60 | 0.60 | Thickener |
| Acrylates/C$_{10-30}$ Alkyl Acrylate Crosspolymer | 0.25 | 0.25 | 0.25 | 0.25 | 0.25 | Emulsion stabiliser, Thickener |
| Diisopropyl Adipate | 4.00 | 4.00 | 4.00 | 4.00 | 4.00 | emollient |
| Cyclomethicone & Trimethylsiloxysilicate | 2.00 | 2.00 | 2.00 | 2.00 | 2.00 | Skin conditioner - occlusive |
| Panthenol | 0.50 | 0.50 | 0.50 | 0.50 | 0.50 | Moisturiser |
| Tocopheryl Acetate | 0.50 | 0.50 | 0.50 | 0.50 | 0.50 | Anti-inflammatory, antioxidant |
| Phytantriol | 0.25 | 0.25 | 0.25 | 0.25 | 0.25 | Moisturiser, Deposition enhancer |
| Propylene Glycol | 5.00 | 5.00 | 5.00 | 5.00 | 5.00 | Humectant |
| Phenoxyethanol and Parabens | 1.00 | 1.00 | 1.00 | 1.00 | 1.00 | Preservative |
| Hydrolysed Soy Protein | 3.00 | | | | | Oil control |
| Hydrolysed Wheat Protein | | 3.00 | | | | Oil control |
| Cedarwood | | | | 3.00 | | Oil control |
| Poplar Bud | | | 3.00 | | | Oil control |
| Dichlorophenyl Imidazoldioxolan | | | | | 0.10 | Oil control |
| Triethanolamine | Qs | Qs | Qs | Qs | Qs | Neutraliser |
| Fragrance | 0.05 | 0.05 | 0.05 | 0.05 | 0.05 | Fragrance |

*EXAMPLE 2*

*Gel Compositions of the invention*

**PROCESS**

<u>Phase A (Main Batch)</u>

1. Add **Purified Water** to mixing vessel.
2. Sprinkle in **PVM/MA Decadiene Crosspolymer** and mix well until dispersed.
3. Start heating to 70-75°C whilst mixing. At 75 - 80°C, sprinkle in **Acrylates C10 -30 Alkyl Acrylate Crosspolymer.** Mix until dispersed.
4. Then add **Panthenol** and continue to mix.
5. Hold at 75 - 80°C for phasing.

<u>Phase B (Oil Phase)</u>

6. To separate vessel add **Diisopropyl Adipate** + **Cyclomethicone & Trimethylsiloxysilicate**. Mix and heat to 75 - 80°C.

<u>Phasing</u>

7. Transfer the Water phase to the Silverson.
8. When the Oil Phase and the Water phase is at 75 - 80°C, <u>slowly,</u> add the Oil phase to the water phase. Homogenizing for 5 minutes to ensure a uniform batch.
9. Add **Triethanolamine** slowly and mix for 5 minutes or until a homogeneous batch is achieved.
10. Turn off homogenizer and mix and cool to 50°C.
11. Add the following premixes for the following compositions:
#1: **Hydrolysed Soy Protein + Propylene Glycol + Phytantriol**
#2: **Hydrolysed Wheat protein + Propylene Glycol + Phytantriol**
#3: **Poplar Bud + Propylene Glycol + Phytantriol**
#4: **Cedarwood + Propylene glycol + Phytantriol**
#5: **Dichlorophenyl Imidazoldioxolan + Propylene Glycol + Phytanatriol**
12. At below 50°C, **Phenoxyethanol and Parabens, Fragrance** and **Tocopheryl Acetate.** Mix for
20 - 25 minutes or until uniform.
13. Viscosity should normally be between 10,000 and 55,000 cpa and pH should be between 5.5 and 6.5, both being measured at 25°C.

## EXAMPLES 3 to 6
### (Gels)

| CTFA Name | Composition 3 Hydrolysed Soy Protein % w/w | Composition 4 Hydrolysed Wheat Protein % w/w | Composition 5 Poplar Bud % w/w | Composition 6 Cedarwood % w/w | Function |
|---|---|---|---|---|---|
| Purified Water | Qs | Qs | Qs | Qs | vehicle |
| PVM/MA Decadiene Crosspolymer | 0.60 | 0.60 | 0.60 | 0.60 | Thickener |
| Acrylates/$C_{10-30}$ Alkyl Acrylate Crosspolymer | 0.25 | 0.25 | 0.25 | 0.25 | Emulsion stabiliser, Thickener |
| Diisopropyl adipate/Isohexadecane | 4.00 | 4.00 | 4.00 | 4.00 | emollient |
| Cyclomethicone & Trimethylsiloxysilicate | 2.00 | 2.00 | 2.00 | 2.00 | Skin conditioner - occlusive |
| Panthenol | 0.50 | 0.50 | 0.50 | 0.50 | Moisturiser |
| Tocopheryl Acetate | 0.50 | 0.50 | 0.50 | 0.50 | ,Antioxidant |
| *Allantoin / Alpha Bisabolol* | *0.20* | *0.20* | *0.20* | *0.20* | *Anti-inflammatory* |
| Phytantriol | 0.25 | 0.25 | 0.25 | 0.25 | Moisturiser, Deposition enhancer |
| Propylene Glycol | 5.00 | 5.00 | 5.00 | 5.00 | Humectant |
| *Salicylic Acid* | *0.50* | *0.50* | *0.50* | *0.50* | *Keratolytic agent* |
| Phenoxyethanol and Parabens | 1.00 | 1.00 | 1.00 | 1.00 | Preservative |
| Hydrolysed Soy Protein | 3.00 | | | | Oil control |
| Hydrolysed Wheat Protein | | 3.00 | | | Oil control |
| Cedarwood | | | | 3.00 | Oil control |
| Poplar Bud | | | 3.00 | | Oil control |
| Triethanolamine/ Sodium Hydroxide | qs | Qs | Qs | Qs | Neutraliser |
| Fragrance | 0.05 | 0.05 | 0.05 | 0.05 | Fragrance |

EP 1 172 087 A2

## EXAMPLES 7 to 10

### (Lotions)

| CTFA Name | Composition 7 Hydrolysed Soy Protein % w/w | Composition 8 Hydrolysed Wheat Protein % w/w | Composition 9 Poplar Bud % w/w | Composition 10 Cedarwood % w/w | Function |
|---|---|---|---|---|---|
| Purified Water | Qs | Qs | Qs | Qs | vehicle |
| Carbomer | 0.20 | 0.20 | 0.20 | 0.20 | Thickener |
| Acrylates/$C_{10-30}$ Alkyl Acrylate Crosspolymer | 0.20 | 0.20 | 0.20 | 0.20 | Emulsion stabiliser, Thickener |
| C12-15 Alkyl Benzoate | 2.50 | 2.50 | 2.50 | 2.50 | emollient |
| Dimethicone | 0.50 | 0.50 | 0.50 | 0.50 | Skin conditioner - occlusive |
| Cyclomethicone | 1.00 | 1.00 | 1.00 | 1.00 | Skin conditioner - emollient |
| Allantoin | 0.20 | 0.20 | 0.20 | 0.20 | Anti-inflammatory |
| Cetyl Alcohol | 1.50 | 1.50 | 1.50 | 1.50 | Co-emulsifier |
| Stearyl Alcohol | 1.00 | 1.00 | 1.00 | 1.00 | Co- emulsifier |
| Stearic Acid | 0.75 | 0.75 | 0.75 | 0.75 | Emulsifier |
| Glyceryl Stearate | 0.50 | 0.50 | 0.50 | 0.50 | Emulsifier |
| Propylene Glycol Isostearoyl | 0.75 | 0.75 | 0.75 | 0.75 | Emulsifier |
| Sodium Isostearoyl Lactylate | 0.25 | 0.25 | 0.25 | 0.25 | Emulsifier |
| Polyoxyethylene (100) Stearate | 0.35 | 0.35 | 0.35 | 0.35 | Emulsifier |
| Propylene Glycol | 3.00 | 3.00 | 3.00 | 3.00 | Humectant |
| Salicylic Acid | 0.50 | 0.50 | 0.50 | 0.50 | Keratolytic agent |
| Phenoxyethanol and Parabens | 1.00 | 1.00 | 1.00 | 1.00 | Preservative |
| Hydrolysed Soy Protein | 0.50 - 3.00 | | | | Oil control |
| Hydrolysed Wheat Protein | | 0.50 - 3.00 | | | Oil control |
| Cedarwood | | | | 0.50 - 3.00 | Oil control |
| Poplar Bud | | | 0.50 - 3.00 | | Oil control |
| Sodium Hydroxide | qs | Qs | Qs | Qs | Neutraliser |
| Fragrance | 0.08 | 0.08 | 0.08 | 0.08 | Fragrance |

EP 1 172 087 A2

## EXAMPLES 11 to 14

### (Lotions)

| CTFA Name | Composition 11 Hydrolysed Soy Protein % w/w | Composition 12 Hydrolysed Wheat Protein % w/w | Composition 13 Poplar Bud % w/w | Composition 14 Cedarwood % w/w | Function |
|---|---|---|---|---|---|
| Purified Water | Qs | Qs | Qs | Qs | vehicle |
| Acrylates/C$_{10-30}$ Alkyl Acrylate Crosspolymer | 0.20 | 0.20 | 0.20 | 0.20 | Emulsion stabiliser, Thickener |
| Disodium EDTA | 0.05 | 0.05 | 0.05 | 0.05 | Chelating agent |
| Dimethicone | 0.50 | 0.50 | 0.50 | 0.50 | Skin conditioner – occlusive |
| BHT | 0.02 | 0.02 | 0.02 | 0.02 | Antioxidant |
| Cetyl Alcohol | 1.50 | 1.50 | 1.50 | 1.50 | Co - emulsifier |
| Glyceryl Monostearate | 1.50 | 1.50 | 1.50 | 1.50 | Co-emulsifier |
| Allantoin | 0.20 | 0.20 | 0.20 | 0.20 | Anti-inflammatory |
| Stearic Acid | 1.00 | 1.00 | 1.00 | 1.00 | Emulsifier |
| Propylene Glycol | 3.00 | 3.00 | 3.00 | 3.00 | Humectant |
| Salicylic Acid | 0.50 | 0.50 | 0.50 | 0.50 | Keratolytic agent |
| Aluminium Starch Octenyl-succinate | 1.50 | 1.50 | 1.50 | 1.50 | Detackifier |
| Phenoxyethanol and Parabens | 1.00 | 1.00 | 1.00 | 1.00 | Preservative |
| Hydrolysed Soy Protein | 0.50 - 3.00 | | | | Oil control |
| Hydrolysed Wheat Protein | | 0.50 - 3.00 | | | Oil control |
| Cedarwood | | | | 0.50 - 3.00 | Oil control |
| Poplar Bud | | | 0.50 - 3.00 | | Oil control |
| Sodium Hydroxide | Qs | Qs | Qs | Qs | Neutraliser |
| Fragrance | 0.08 | 0.08 | 0.08 | 0.08 | Fragrance |

# EXAMPLES 15 to 18

## (Lotions)

| CTFA Name | Composition 15 Hydrolysed Soy Protein % w/w | Composition 16 Hydrolysed Wheat Protein % w/w | Composition 17 Poplar Bud % w/w | Composition 18 Cedarwood % w/w | Function |
|---|---|---|---|---|---|
| Purified Water | Qs | Qs | Qs | Qs | vehicle |
| PVM/MA Decadiene Crosspolymer | 0.40 | 0.40 | 0.40 | 0.40 | Thickener |
| Acrylates/$C_{10-30}$ Alkyl Acrylate Crosspolymer | 0.20 | 0.20 | 0.20 | 0.20 | Emulsion stabiliser, Thickener |
| C12-15 AlkylBenzoate | 2.50 | 2.50 | 2.50 | 2.50 | emollient |
| Cyclomethicone & Trimethylsiloxysilicate | 2.00 | 2.00 | 2.00 | 2.00 | Skin conditioner - occlusive |
| Disodium EDTA | 0.50 | 0.50 | 0.50 | 0.50 | Chelating Agent |
| Cetyl Alcohol | 0.50 | 0.50 | 0.50 | 0.50 | Co-emulsifier |
| Allantoin | 0.20 | 0.20 | 0.20 | 0.20 | Anti-inflammatory |
| Aluminium Starch Octenylsuccinate | 1.20 | 1.20 | 1.20 | 1.20 | Detackifier |
| Propylene Glycol | 5.00 | 5.00 | 5.00 | 5.00 | Humectant |
| Salicylic Acid | 0.50 | 0.50 | 0.50 | 0.50 | Keratolytic agent |
| Phenoxyethanol and Parabens | 1.00 | 1.00 | 1.00 | 1.00 | Preservative |
| BHT | 0.02 | 0.02 | 0.02 | 0.02 | Antioxidant |
| Hydrolysed Soy Protein | 0.50 - 3.00 | | | | Oil control |
| Hydrolysed Wheat Protein | | 0.50 - 3.00 | | | Oil control |
| Cedarwood | | | | 0.50 - 3.00 | Oil control |
| Poplar Bud | | | 0.50 - 3.00 | | Oil control |
| Sodium Hydroxide | Qs | Qs | Qs | Qs | Neutraliser |
| Fragrance | 0.08 | 0.08 | 0.08 | 0.08 | Fragrance |

## EXAMPLES 19 to 22

### (Creams)

| CTFA Name | Composition 19 Hydrolysed Soy Protein % w/w | Composition 20 Hydrolysed Wheat Protein % w/w | Composition 21 Poplar Bud % w/w | Composition 22 Cedarwood % w/w | Function |
|---|---|---|---|---|---|
| Purified Water | Qs | Qs | Qs | Qs | vehicle |
| Acrylates/$C_{10-30}$ Alkyl Acrylate Crosspolymer | 0.15 | 0.15 | 0.15 | 0.15 | Emulsion stabiliser, Thickener |
| Carbomer | 0.30 | 0.30 | 0.30 | 0.30 | Thickener |
| Disodium EDTA | 0.05 | 0.05 | 0.05 | 0.05 | Chelating agent |
| Dimethicone | 0.50 | 0.50 | 0.50 | 0.50 | Skin conditioner - occlusive |
| BHT | 0.02 | 0.02 | 0.02 | 0.02 | Antioxidant |
| Cetyl Alcohol | 1.50 | 1.50 | 1.50 | 1.50 | Co - emulsifier |
| Glyceryl Monostearate | 1.50 | 1.50 | 1.50 | 1.50 | Co-emulsifier |
| Allantoin | 0.20 | 0.20 | 0.20 | 0.20 | Anti-inflammatory |
| Stearic Acid | 1.00 | 1.00 | 1.00 | 1.00 | Emulsifier |
| Propylene Glycol | 3.00 | 3.00 | 3.00 | 3.00 | Humectant |
| Salicylic Acid | 0.50 | 0.50 | 0.50 | 0.50 | Keratolytic agent |
| Aluminium Starch Octenyl-succinate | 1.50 | 1.50 | 1.50 | 1.50 | Detackifier |
| Phenoxyethanol and Parabens | 1.00 | 1.00 | 1.00 | 1.00 | Preservative |
| Hydrolysed Soy Protein | 0.50 - 3.00 | | | | Oil control |
| Hydrolysed Wheat Protein | | 0.50 - 3.00 | | | Oil control |
| Cedarwood | | | | 0.50 - 3.00 | Oil control |
| Poplar Bud | | | 0.50 - 3.00 | | Oil control |
| Sodium Hydroxide | Qs | Qs | Qs | Qs | Neutraliser |
| Fragrance | 0.08 | 0.08 | 0.08 | 0.08 | Fragrance |

## EXAMPLES 23 to 26
### (Conditioning Liquids)

| CTFA Name | Composition 23 Hydrolysed Soy Protein % w/w | Composition 24 Hydrolysed Wheat Protein % w/w | Composition 25 Poplar Bud % w/w | Composition 26 Cedarwood % w/w | Function |
|---|---|---|---|---|---|
| Water | Qs | Qs | Qs | Qs | Vehicle |
| Butylene Glycol | 2.00 | 2.00 | 2.00 | 2.00 | Solvent |
| Salicylic acid | 0.50 | 0.50 | 0.50 | 0.50 | Keratolytic agent |
| Sodium Hydroxide | 1.50 | 1.50 | 1.50 | 1.50 | Neutraliser |
| Glycereth-26 | 1.00 | 1.00 | 1.00 | 1.00 | Skin conditioner |
| Polyquaternium –7 | 0.15 | 0.15 | 0.15 | 0.15 | Skin conditioner, film former |
| Phenoxyethanol & Parabens | 0.25 | 0.25 | 0.25 | 0.25 | Preservative |
| Polysorbate 20 | 0.28 | 0.28 | 0.28 | 0.28 | Solubiliser |
| Fragrance | 0.015 | 0.015 | 0.015 | 0.015 | Fragrance |
| Allantoin | 0.20 | 0.20 | 0.20 | 0.20 | Anti-inflammatory |
| Hydrolyzed Soy Protein | 0.50 – 3.00 | | | | Oil Control |
| Hydrolysed Wheat Protein | | 0.50 – 3.00 | | | Oil Control |
| Poplar Bud | | | 0.50 – 3.00 | | Oil Control |
| Cedarwood | | | | 0.50 – 3.00 | Oil Control |
| Panthenol | 0.25 | 0.25 | 0.25 | 0.25 | Moisturiser |

## EXAMPLES 27 to 30

### (Cleansers)

| CTFA Name | Composition 27 Hydrolysed Soy Protein % w/w | Composition 28 Hydrolysed Wheat Protein % w/w | Composition 29 Poplar Bud % w/w | Composition 30 Cedarwood % w/w | Function |
|---|---|---|---|---|---|
| Water | Qs | Qs | Qs | Qs | Vehicle |
| Triclosan | 0.25 | 0.25 | 0.25 | 0.25 | Antibacterial agent |
| Lauric acid | 4.30 | 4.30 | 4.30 | 4.30 | Cleansing |
| Myristic acid | 5.30 | 5.30 | 5.30 | 5.30 | Cleansing |
| Lauryl Phosphate | 4.00 | 4.00 | 4.00 | 4.00 | Surfactant |
| Hydroxypropyl Methylcellulose | 0.85 | 0.85 | 0.85 | 0.85 | Thickener |
| Triethanolamine | 17.00 | 17.00 | 17.00 | 17.00 | Neutraliser |
| BHT | *0.02* | *0.02* | *0.02* | *0.02* | *Antioxidant* |
| Sodium Citrate | 0.20 | 0.20 | 0.20 | 0.20 | Chelating agent |
| Glycerin | 8.00 | 8.00 | 8.00 | 8.00 | Humectant |
| Cocamido Propyl Betaine | 3.00 | 3.00 | 3.00 | 3.00 | Foam Booster |
| Phenoxyethanol & Parabens | 1.00 | 1.00 | 1.00 | 1.00 | Preservative |
| Hydrolysed Soy Protein | 0.50- 3.00 | | | | Oil Control |
| Hydrolysed Wheat Protein | | 0.50 -3.00 | | | Oil Control |
| Poplar Bud | | | 0.50 - 3.00 | | Oil Control |
| Cedarwood | | | | 0.50 - 3.00 | Oil Control |
| Fragrance | 0.30 | 0.30 | 0.30 | 0.30 | Fragrance |

14

## EXAMPLE 31

| Formulation/Composition Information | | | | |
|---|---|---|---|---|
| Component CTFA/ Technical Name | Function | %Active | %(w/w) | %(w/w) Active |
| Butylene Glycol | Humectant | 100 | 2.00 | 2.00 |
| Salicylic Acid | Keratolytic | 100 | 0.50 | 0.50 |
| Sodium Hydroxide | pH adjustment | 10 | 1.50 | 0.15 |
| Glycereth-26 | Skin Conditioner | 100 | 1.00 | 1.00 |
| Polyquaternium-7 | Skin Conditioner | 8 | 0.15 | 0.01 |
| Methyl Paraben(and)Ethyl Paraben(and)Propyl Paraben(and)Butyl Paraben(and)Phenoxyethanol | Perservative | 100 | 0.25 | 0.25 |
| Polysorbate 20 | Solubliser | 100 | 0.28 | 0.28 |
| Hydrolysed Soy Protein(and)Propylene glycol | Oil Control Active | 100 | 1.50 | 1.50 |
| Fragrance | Fragrance | 100 | 0.02 | 0.02 |
| Allantoin | Anti-inflammatory | 100 | 0.20 | 0.20 |
| D-Panthenol | Skin Conditioner | 100 | 0.25 | 0.25 |
| Water | Vehicle | 100 | 92.36 | 92.36 |
| Total | | | 100 | |

## EXAMPLE 32

An evaluation of the sebum-reducing activity of moisturizing gels containing Hydrolyzed Wheat Protein and Hydrolyzed Soy Protein were tested against similar gels containing elubiol on Philippine citizens.

35 teenagers (19 male, 16 female) between the ages of 13 and 17, with oily skin (sebum readings above 180 $\mu g/cm^2$ on the forehead/nose when measured with the Sebumeter SM 810), in good health and with no apparent epidermal irregularities, participated in this study. To assure uniform test parameters, all panellists were pre-screened by the dermatologist at the test center. Only 29 respondents (14 used Hydrolyzed Wheat Protein vs. Elubiol, 15 used Hydrolyzed Soy Protein vs. Elubiol) were able to complete the study because 6 voluntarily discontinued.

Each subject was supplied with Clean & Clear Facial Wash two week prior to the commencement of the study, serving as a wash out/conditioning period. During the study proper, the subject washed his/her face with the supplied facial wash. After drying the face thoroughly, he/she then applied the product designated as LEFT on the left side of his/her face using the left forefinger, then applied the product designated as RIGHT on the right side of his/her face using the right forefinger. Product assignment per subject and side of the face was randomized. Each subject used one test product and the control. Product application was done twice a day for a period of 12 weeks. Instrumental evaluations were made on weeks 0, 3, 6, 9 and 12.

Instrumental measurements using Sebumeter SM 810 were performed in a temperature and humidity controlled environment. The temperature was maintained at 25-28°C and humidity within 40-60% range. These conditions were recorded during evaluation days. Subjects were instructed not to drink hot caffeinated drinks 1 hour before evaluation and were required to acclimatize to room conditions for at least 10 minutes prior to measurements.

Sebum readings were taken by pressing the matted plastic film of the cassette with a force of 4N for 20 seconds on a designated area of the face. The skin area measured was approximately 64 $mm^2$. The cassette was then inserted into the aperture of the Sebumeter. The sebum absorbed by the film was analyzed by photometry, and the sebum reading in $\mu g/cm^2$ was then displayed and recorded. Two readings were taken on each of these test sites: left forehead, left cheek, right forehead and right cheek.

Since the study was conducted during the colder months of the year, the sebum reading minimum requirement was set at 180 $\mu g/cm^2$, in order to meet the quota for the number of subjects.

Percentage sebum reduction was computed by subtracting subsequent timepoint readings from baseline reading and dividing the difference by the baseline reading. Analysis of variance was then performed on the percentage sebum reduction during weeks 3, 6, 9 and 12, with $p \leq 0.05$ used as criterion of significance.

Mean sebum readings charts showed Hydrolyzed Wheat Protein and Hydrolyzed Soy Protein exhibiting similar profiles versus Elubiol, with sebum readings decreasing with time, from 124-138 $\mu g/cm^2$ at baseline to 68-81 $\mu g/cm^2$ at week 12.

## EXAMPLE 33

In a similar trial in Australia on Australian teenagers, using a sebum reading minimum requirement of 220 $\mu g/cm^2$, again showed Hydrolyzed Wheat Protein and Hydrolyzed Soy Protein exhibiting similar profiles to elubiol with sebum readings decreasing from 145-155 $\mu g/cm^2$ at baseline to 117-124 $\mu g/cm^2$ at week 12.

## EXAMPLE 34

A study was conducted on 37 normal volunteers in India to determine the oil reduction potential of moisturising gels containing sebum reduction agents.

This study was a blinded, randomised study comparing 2 test gels (containing Cedar wood and Poplar bud) with standard preparation (containing Elubiol).

Women volunteers between the age of 17 and 19 years were screened by measuring sebumeter readings of the left forehead using a Sebumeter SM810. Those volunteers had subumeter reading of 180 $\mu m/cm^2$ or above.

These volunteers were instructed to avoid any other face cream, face wash or any other cosmetics during the 8 weeks of the study. After recruitment, volunteers were instructed to use the face wash for the first 2 weeks. This period was considered as the conditioning period.

A baseline sebumeter reading was taken at the end of the conditioning period on 4 sites i.e. left forehead, left cheek, right forehead, and right cheek. Tubes containing the test medicines (which were coded and randomly allocated to each site) were dispensed to the volunteers. Each volunteer was given two test gels, one for left side of the face and the other for right side of the face. The volunteers were instructed to apply the test gels twice a day after washing the face with the given face wash for next 6 weeks.

The volunteer was requested to make a note of application of face wash and face cream daily on the given diary sheets. Sebumeter readings were taken 3 weeks and 6 weeks later.

Cedar wood and elubiol application led to a significantly greater fall in the sebum reading at 6 weeks as compared to 3 weeks. Treatment with Poplar bud, on the other hand showed no difference between 3rd and 6th week.

A comparison between the treatments indicated that at 3 weeks the average percentage reduction in sebumeter readings on forehead and cheek was best with cedarwood (p<0.01 better than elubiol). The effect of poplar bud was comparable to elubiol.

At 6 weeks the effects of cedarwood were consistently maintained. The effect of elubiol improved, while poplar bud remained the least effective although this was not a consistent effect as seen by the wide variation in response.

## EXAMPLE 35

In this example the sebum control properties of moisturizing gels with plant extracts, cedarwood extract and hydrolysed soy protein, at a lower concentration of 0.5% is demonstrated.

This study was a double-blind, randomised, single centre study comparing 2 test gels (containing hydrolysed soy protein and cedarwood extract) with placebo

### Test products:

| Product | Active |
| --- | --- |
| MT | Cedarwood extract - 0.5% |
| DE | Hydrolysed soy protein - 0.5% |
| BO | Placebo. |

40 female volunteers aged between 13 to 19 years and having a sebumeter reading of 180$\mu g/cm^2$ took part in the study. These volunteers were instructed to avoid any other face cream, face wash or any other cosmetics during the 12 weeks of the study. During the first 2 weeks of the study, volunteers were instructed to use the face wash twice a day for washing their face. This period was considered as the conditioning period.

A baseline sebumeter reading was taken at the end of the conditioning period on 4 sites i.e. left forehead, left cheek, right forehead, and right cheek. Tubes containing the test products (which were coded and randomly allocated to each site) were dispensed to the volunteers. Each volunteer was given two test gels, one for left side of the face and the other for right side of the face. The choice of gel for the specific side of the face was made by random allocation. The volunteers were instructed to apply the test gels twice a day after washing the face with the given face wash for the next 12 weeks. Both volunteer and the study coordinator were not aware of the treatment dispensed ensuring the double blind nature of the study.

Adverse events were recorded.

## Results:

The two test products significantly reduced sebum readings as compared to the placebo.

The average % reduction for each of the three gels is tabulated below:

Table 1 (a) -

| % Reduction (forehead) | | | | |
|---|---|---|---|---|
| Treatment | Time (weeks) | | | |
| | 3 | 6 | 9 | 12 |
| HSP | 16.84 | 41.09 | 61.14 | 63.41 |
| Placebo | 14.1 | 23.41 | 29.97 | 39.62 |

Table 1 (b) -

| % Reduction (forehead) | | | | |
|---|---|---|---|---|
| Treatment | Time (weeks) | | | |
| | 3 | 6 | 9 | 12 |
| HSP | 19.17 | 35.94 | 48.79 | 56.23 |
| Placebo | 11.89 | 23.19 | 35.09 | 46.02 |

Table 2 (a) -

| % Reduction (forehead) | | | | |
|---|---|---|---|---|
| Treatment | Time (weeks) | | | |
| | 3 | 6 | 9 | 12 |
| HSP | 19.17 | 35.94 | 48.79 | 56.23 |
| Placebo | 11.89 | 23.19 | 35.09 | 46.02 |

Table 2(b) -

| % Reduction (cheek) | | | | |
|---|---|---|---|---|
| Treatment | Time (weeks) | | | |
| | 3 | 6 | 9 | 12 |
| HSP | 20.28 | 47.05 | 58.68 | 68.11 |
| Placebo | 17.91 | 32.57 | 43.51 | 51.81 |

Table 3(a) -

| % Reduction (Forehead+ cheek) | | | | |
|---|---|---|---|---|
| Treatment | Time (weeks) | | | |
| | 3 | 6 | 9 | 12 |
| cw | 18.21 | 40.76 | 59.7 | 62.45 |
| Placebo | 13.12 | 24.99 | 33.4 | 42.45 |

Table 3(b) -

| % Reduction (Forehead+ cheek) | | | | |
|---|---|---|---|---|
| Treatment | Time (weeks) | | | |
| | 3 | 6. | 9 | 12 |
| HSP | 19.7 | 41.23 | 53.5 | 61.88 |
| Placebo | 14.8 | 27.73 | 39.16 | 48.82 |

Table 4.:

| Reduction in sebum levels for the three gels at the end of 12 weeks. | | | | | |
|---|---|---|---|---|---|
| Product | 0-3 | 3-6 | 6-9 | 9-12 | 0-12 |
| | | | | | |
| CW | 0.041 * | 0.006* | 0.004* | 0.066 | 0.00* |
| HSP | 0.011* | 0.0012* | 0.0059* | 0.027* | 0.00* |
| Placebo | 0.06 | 0.12 | 0.23 | 0.18 | 0.00* |

*$P < 0.05$ - Significant

Chart1.

OCS - % reduction (Forehead +Cheek)

Chart2.

OCS- % REDUCTION -FOREHEAD

Chart.3.

OCS -% REDUCTION-CHEEK

Chart 4.

Chart 5.

Chart 6.

OCS - % REDUCTION (FOREHEAD +CHEEK)

Chart 7

OCS - % reduction (Forehead +Cheek)

**Conclusions:**

1) The two extracts- cedarwood and poplar bud extract retained their activity at 0.5%. and effectively reduced the sebum levels.

2) The two extracts performed at parity at the end of the study period.

3) Though the placebo also appeared to lower the sebum level the activity was significantly lower than the test gels.

*EXAMPLE 36*

In this example the lipase inhibition activity of cedarwood extract and hydrolysed soy protein is demonstrated. The test was carried out on female volunteers within the age group of 13-18 years having a sebumetre reading of 180 ug/cm2.

A lipase inhibition kit from Sigma was used for the study. This kit consists of a substrate, trezma buffer, and indicator. Standard lipase was procured separately.

The reaction mixtures and blanks were prepared as listed below:

| Reaction mix. | Substrate | Buffer | Lipase | water | Extract (inhibitor) |
|---|---|---|---|---|---|
| Test Std. | 10ml | 1ml | 1ml | 2.5ml | - |
| Test Mix. . | 10ml | 1ml | 1ml | 2ml | 0.5ml |
| Blank | 10ml | 1ml | - | 3.5ml | - |

The solutions were pippetted out into the test tubes as mentioned above. The tubes were shaken vigorously for around 5 secs. and were kept in a constant temperature bath at 37°C for 3 hours. After the incubation period contents of the test tubes were poured into titration flasks and 3ml of ethanol was pipetted into each flask. Six drops of thymol phenophthalein indicator was added. In a burette 0.05 N NaOH solution was taken and used for titration. Each flask was titrated to a deep blue color which lasted for about 30secs. The blank and the test mixtures needed to show the same blue color. The final reading was noted and the initial reading subtracted from the final. This is the amount of NaOH required to neutralise the fatty acids liberated due to lipase activity.

The activity was calculated as follows:

0.05N NaOH used for test mix.- 0.05N NaOH used for blank = 0.05N NaOH required to neutralise fatty acids liberated.

Lipase activity = Amount of 0.05N NaOH.

**Results:** The burette reading for the test mixtures and the blank are tabulated below:

| Test Mixture | Burette reading | Lipase Activity (Test mix- Blank) |
|---|---|---|
| Lipase | 22 | - |
| Lipase+CW | 15.2 | 12.4 |
| Lipase+HSP | 16.2 | 13.4 |
| Blank | 2.8 | - |
| | | |

Different concentrations of the inhibitor from 0.2-1.0% were used to determine the optimum concentration. The results of these are as given below:

| **Cedarwood** | |
|---|---|
| Concentration (%) | Lipase Activity |
| 0.2 | 19.66 |
| 0.4 | 19.52 |
| 0.6 | **17.84** |
| 0.8 | 19.5 |
| 1 | 20.18 |
| Standard Lipase | 20.3 |

| Hydrolysed soy protein | |
|---|---|
| Concentration (%) | Lipase Activity |
| 0.2 | 19.8 |
| 0.4 | **18.3** |
| 0.6 | 21.0 |
| 0.8 | 21.2 |
| 1.0 | 21.1 |
| Standard Lipase | 22 |

**Conclusion:** The two extracts demonstrated lipase inhibition. The optimum concentrations were 0.4% for Hydrolysed Soy Protein and 0.6% for cedarwood respectively.

Effect of inhibitors on Lipase Activity.

Effect of different concentrations of HSP on Lipase inhibition

EP 1 172 087 A2

Effect of different concnetrations of CW on lipase inhibition

## Conclusion

All three tested drugs showed significant sebum reducing properties.

While this invention has been described with reference to preferred embodiments it is not to be construed as limited thereto. Furthermore where specific features have been mentioned and equivalents exist thereto, such equivalents are included herein as if specifically set forth.

## Claims

1. The use as a sebum regulating agent of a hydrolysed vegetable protein produced by enzymatic hydrolysis.

2. A composition for application to the skin to prevent or inhibit the development of acne or to treat acne when present comprising a hydrolysed vegetable protein produced by enzymatic hydrolysis.

3. A method of preventing, controlling or inhibiting the oily/shiny appearance of skin comprising the topical application of a hydrolysed vegetable protein, produced by enzymatic hydrolysis to the skin.

4. The use composition or method, as claimed in claim 1, 2 or 3 wherein the vegetable protein is a cereal protein such as soy protein.

5. A composition as claimed in claim 2 including other active agents designed to assist in improving skin appearance and assist in inhibiting the development of other conditions such as acne.

6. A composition as claimed in claim 5 wherein the other active is selected from the group of keratolytic agents and anti-inflammatory agents.

7. A composition as claimed in claim 6 wherein the keratolytic agent is salicylic acid, benzoyl peroxide, resorcinol, colloidal sulphur, selenium disulphide or sulfur.

8. A composition as claimed in claim 6 or 7 wherein the anti-inflammatory agent is alpha-bisabolol, dipotassium glycyrrhizinate, allantoin, matricaria (chamomilla recutita) extract, tocopheryl acetate, green tea (camellia sinesis) extract, turmeric (curcuma longa) extract.

9. The use of a hydrolysed cereal protein for controlling, or at least inhibiting the oily/shiny appearance of skin.

10. The use as claimed in claim 9 wherein the protein is wheat protein.

11. A composition for use in inhibiting, or controlling, consequences of oily skin such as acne, comprising a hydrolysed cereal protein, and a suitable carrier.

**12.** A composition as claimed in claim 10 wherein the protein is wheat protein.

**13.** A method of controlling or at least inhibiting, the oily nature of skin, comprising the topical application of a composition containing a hydrolysed cereal protein to the affected part of the skin.

**14.** The use of a hydrolysed cereal protein in the manufacture of a medicament for treating acne.

**15.** The use as claimed in claim 14 wherein the protein is wheat protein.

**16.** The use as claimed in claim 9, 10, 11, 12, 13, 14 or 15 wherein the wheat protein is a soluble wheat protein, preferably of a high molecular weight having a molecular weight in the region of 100,000 to 500,000 Daltons.

**17.** A composition as claimed in claim 11, 12 or 16 including a keratolytic agent and an anti-inflammatory agent.

**18.** A composition as claimed in claim 17 wherein the keratolytic agent is salicylic acid, benzoyl peroxide, resorcinol, colloidal sulphur, selenium disulphide or sulfur.

**19.** A composition as claimed in claim 17 or 18 wherein the anti-inflammatory agent is alpha-bisabolol, dipotassium glycyrrhizinate, allantoin, matricaria (chamomilla recutita) extract, tocopheryl acetate, green tea (camellia sinesis) extract or turmeric (curcuma longa) extract.

**20.** The use for preventing, inhibiting or treating oily skin of a combination of a sebum controlling agent and a keratolytic agent, applied sequentially or simultaneously.

**21.** A composition for the use as claimed in claim 20 including a sebum regulating agent and a keratolytic agent.

**22.** The use of a sebum regulating agent in the manufacture of a medicament for use in a method of inhibiting, treating or preventing acne, said method comprising the application to the affected area of the skin, or the area susceptible to be affected, sequentially or simultaneously of said sebum regulating agent and a keratolytic agent.

**23.** The use or composition as claimed in claim 20, 21 or 22 wherein the sebum regulating agent is a plant extract, such as hydrolysed vegetable protein, such as hydrolysed cereal protein, in particular, hydrolysed wheat or soy protein, or an extract from a tree for example cedar, poplar or mimosa, preferably from the foliage or from the various stages of the flower, such as the bud.

**24.** The use, or composition as claimed in claim 23 wherein the keratolytic agent is benzoyl peroxide, resorcinol, colloidal sulphur, salicylic acid, selenium disulphide or sulfur, preferably salicylic acid.

**25.** A method of controlling the oily/shiny appearance of skin comprising applying to the skin having such appearance or susceptible to such disorder, a lipase inhibiting substance.

**26.** A topical composition for use in the method of claim 25 comprising a lipase inhibitor and suitable carrier.

**27.** A method as claimed in claim 25 further including a keratolyic agent.

**28.** The use of a hydrolysed vegetable protein, produced by enzymatic hydrolysis, in the manufacture of a medicament for the prevention or treatment of skin conditions associated with excessive sebum production, such as acne.